Europäisches Patentamt

European Patent Office

Office européen des brevets

(11) Publication number: **0 094 755**
A2

## (12) EUROPEAN PATENT APPLICATION

(21) Application number: **83302458.1**

(22) Date of filing: **29.04.83**

(51) Int. Cl.³: **A 61 L 15/06**
**A 61 L 15/01, A 61 F 13/02**

(30) Priority: **19.05.82 US 379589**

(43) Date of publication of application:
**23.11.83 Bulletin 83/47**

(84) Designated Contracting States:
**AT BE CH DE FR GB IT LI LU NL SE**

(71) Applicant: **Riaboy, Florence**
**4819 Sorrento Court**
**Cape Coral Florida 33904(US)**

(72) Inventor: **Riaboy, Florence**
**4819 Sorrento Court**
**Cape Coral Florida 33904(US)**

(74) Representative: **Jackson, Peter Arthur et al,**
**GILL JENNINGS & EVERY 53-64 Chancery Lane**
**London WC2A 1HN(GB)**

(54) **Medical dressing.**

(57) A medical dressing for covering skin abrasions or lacerations in the form of a bandaging material (10), preferably a single layer of porous, non-woven polyester, having a pressure-sensitive adhesive on one surface in marginal areas (12) about the periphery thereof, with a central area (14) free of such adhesive. The dressing is provided in pre-cut sizes with a removable sheet of release liner (16) shaped to cover at least the adhesive-coated areas.

FIG. 1

EP 0 094 755 A2

Croydon Printing Company Ltd

FLORENCE RIABOY                    REF.50/2165/02

MEDICAL DRESSING

The present invention relates to self-adhering medical dressings.

Several forms of self-adhering dressings are widely used for protecting minor cuts, bruises and burns from contact with foreign matter to promote healing and lessen the chance of infection. Such dressings generally include an assembly of two or more diverse materials, including a member in strip or other desired form, having a pressure sensitive adhesive on one surface, and a gauze pad or other dressing material, the adhesive strip normally being non-porous, covering the material and extending beyond the boundaries thereof to adhere to the skin and maintain the material in contact with the afflicted area. The adhesive areas are normally covered by a release liner and the assembly may be further packaged in an individual paper envelope. Although certain economies may be realized by automated mass production of such dressings, assuming a large initial investment in equipment, the assembly of a number of diverse materials will obviously involve significant expense.

In other surgical applications, where the use of ready-made, self-adhering dressings is inconvenient or inappropriate, often due to the necessity of covering a larger area, it is the usual practice to use a piece of gauze or other dressing material of appropriate size and securing it to the patient by strips of adhesive tape cut from a separate roll. This is an operation which obviously is more time consuming and requires greater care in accplication than a self-adhering dressing, as well as requiring the immediate availability of two separate items, i.e., the dressing material and the tape.

It is a principal object of the present invention to provide a medical dressing which has the advantage of being self-adhering, but yet is of low cost.

In accordance with the invention, a medical dressing for covering and protecting an afflicted area on the surface of the human body comprises a dressing material having a peripheral boundary of predetermined size and shape; a pressure sensitive adhesive carried upon one surface of the material in marginal areas adjoining and surrounding the boundary, leaving a substantial area of the one surface free of the adhesive; and a layer of release liner of size and shape at least sufficient to cover the adhesive-coated marginal areas and releasably adhered to the adhesive.

A dressing of this construction requires minimum assembly. It is essentially of no greater cost or complexity than an ordinary gauze pad secured by adhesive tape strips, but may be applied more quickly and with far greater convenience. Furthermore, it may be such that only porous material covers the afflicted area, allowing air to pass through and aid in the healing process.

Preferably, the release liner is of size and shape substantially equal to that of the dressing material and is superposed over and completely covers the one surface.

An example of a dressing in accordance with the invention is illustrated in the accompanying drawings, in which:-

Figure 1 is a front elevation with a release liner layer partly removed; and,

Figure 2 is a section taken on the line 2-2 in Figure 1.

The illustrated dressing comprises a layer of dressing material 10 of a type suitable for direct contact with open cuts, burns, abrasions, incisions or other such afflicted areas of the human body, and is preferably a single layer of porous, non-woven fabric of polyester, rayon, cotton or other synthetic or natural material. The particular material is chosen to provide the degree of porosity, absorbency, thickness and other physical attributes appropriate to the general or specific medical applications contemplated.

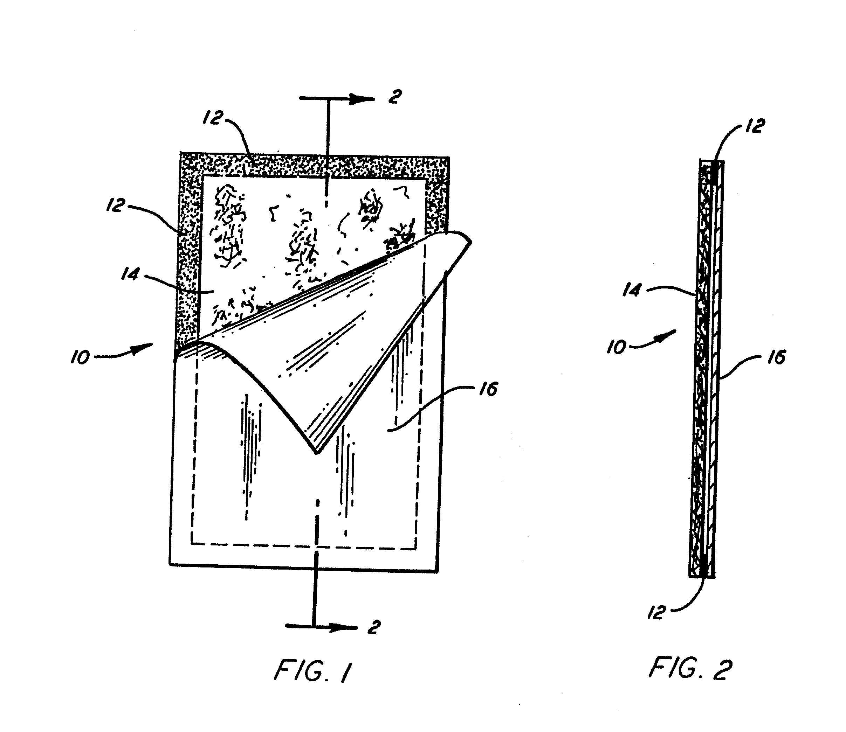

The surface of material 10 partially seen in Figure 1 includes marginal areas 12 around the peripheral border coated with pressure sensitive adhesive of any conventional type which is normally tacky and adheres well to the human skin when pressed thereon. Material 10 is cut to a rectangular shape as shown in Figure 1 but may, of course, be provided in any desired peripheral shape and size.

Surface area 14, within the adhesively coated areas 12, is in covering relation to the afflicted area when the dressing is in use and may be in direct contact with open cuts or other breaks in the skin. Accordingly, it is desirable to protect area 14 from contamination during handling and at all times prior to use. For this purpose, as well as to protect adhesively coated areas 12 and prevent the dressing from sticking to other items prior to use, a sheet of protective material 16 is cut to the same peripheral dimensions as material 10 and placed in superposed relation to surface areas 12 and 14. Material 16 is preferably plastics or a paper base having a waxy coating on one or both sides, or which has been silicone treated, of the type commonly known as release liner which will adhere to adhesive areas 12 but may be readily peeled away, as indicated in Figure 1, without adversely effecting the adhesive properties thereof.

The very simple and inexpensive medical dressing

4

is effective and easy to apply. The dressing material and release liner may, of course, be sterilized and/or further packaged in a paper envelope, or the like, if desired. When this is done, the necessity of covering the surface area 14 is not as great and the release liner may be shaped to cover only adhesive areas 12 rather than the entire surface of the bandage

It is also to be understood that although the material 10 has been described as a single layer of material, a plurality of initially separate layers e.g., of gauze or other materials, may be superposed and joined by heat sealing, needling, or other means, to provide a unitary dressing material having the adhesive coated areas around the periphery of one surface.

## CLAIMS

1. A medical dressing for covering and protecting an afflicted area on the surface of the human body, the dressing comprising a dressing material (10) having a peripheral boundary of predetermined size and shape; a pressure sensitive adhesive (12) carried upon one surface of the material in marginal areas adjoining and surrounding the boundary, leaving a substantial area of the one surface free of the adhesive; and a layer of release liner (16) of size and shape at least sufficient to cover the adhesive-coated marginal areas and releasably adhered to the adhesive.

2. A dressing according to claim 1, wherein the material (10) is a single layer.

3. A dressing according to claim 1 or claim 2 wherein the material (10) is a porous, non-woven material.

4. A dressing according to claim 3, wherein the material (10) is polyester.

5. A dressing according to any one of the preceding claims, wherein the release liner (16) is of size and shape substantially equal to that of the dressing material (10) and is superposed over and completely covers the one surface.

FIG. 1

FIG. 2